# EUROPÄISCHE PATENTSCHRIFT

(11) **EP 0 731 695 B1**
(45) Veröffentlichungstag und Bekanntmachung des Hinweises auf die Patenterteilung: **06.05.1998**
(21) Anmeldenummer: 95901413.5
(22) Anmeldetag: 23.11.1994
(51) Int. Cl.: A61K 9/70

(54) **WIRKSTOFFHALTIGES PFLASTER**
PFLASTER CONTAINING AN ACTIVE SUBSTANCE
EMPLATRE CONTENANT UN PRINCIPE ACTIF

(30) Priorität: 04.12.1993 DE 4341444
(43) Veröffentlichungstag der Anmeldung: 18.09.1996
(73) Patentinhaber: LTS LOHMANN Therapie-Systeme GmbH, 56567 Neuwied (DE)
(72) Erfinder: MECONI, Reinhold, D-56567 Neuwied (DE); SEIBERTZ, Frank, D-53557 Bad Hönningen/Ariendorf (DE)
(74) Vertreter: Flaccus, Rolf-Dieter, Dr.
(86) Internationale Anmeldenummer: EP9403866
(87) Internationale Veröffentlichungsnummer: WO9515158

(56) Entgegenhaltungen:
- EP-A- 0 033 615
- EP-A- 0 205 974
- EP-A- 0 249 475
- WO-A-93/03692
- US-A- 4 963 261

## Beschreibung

Die Erfindung betrifft ein wirkstoffhaltiges Pflaster zur kontrollierten Abgabe von Wirkstoffen mit einer bei Körpertemperatur erweichenden wirkstoffhaltigen Reservoirmasse sowie ein Verfahren zu seiner Herstellung.

Wirkstoffhaltige Pflaster der genannten Art sind bekannt.

Die DE 35 03 111 beschreibt ein Pflaster, das die Arzneistoffe in einer als Scheibe ausgebildeten festen Reservoirmasse enthält, die bei Körpertemperatur erweicht. Die Wirkstofffreisetzung beginnt unmittelbar nach der Applikation.

Ein weiteres Pflaster dieser Art ist in der DE 35 22 060 beschrieben, wobei eine Reservoirmassenscheibe an eine elastische Kunststoffscheibe gleichen Durchmessers fixiert ist. Beide miteinander verbundenen Teile befinden sich in einem auf der Haut abgewandten Seite abgeschlossenen, zur Hautfläche aber offenen und auf der Haut fixierbaren Gehäuse. Die elastische Kunststoffscheibe übt auf die Reservoirschicht einen ständigen Druck aus. Auch hier beginnt die Wirkstofffreisetzung unmittelbar nach Applikation.

Der Beginn der Wirkstofffreisetzung unmittelbar nach Applikation eines Pflasters ist jedoch nicht in allen Fällen erwünscht. So kann aus medizinischer Sicht die Forderung bestehen, ein Pflaster herzustellen, bei dem der Beginn der Wirkstofffreisetzung erst nach einer definierten Zeitspanne nach Applikation eintritt. Es wird damit gewährleistet, daß nur ein Teil der Tragedauer des Pflasters zur Wirkstoffabgabe zur Verfügung steht. Bei täglichem Wechsel des Pflasters wechseln sich somit Phasen ab, in denen kein Wirkstoff freigesetzt oder doch Wirkstoff dem Körper zugeführt wird. Ein Pflaster dieser Art ist aus dem Stand der Technik nicht bekannt.

Es ist somit Aufgabe der Erfindung, ein wirkstoffhaltiges Pflaster mit einer bei Körpertemperatur erweichenden, wirkstoffhaltigen Reservoirmasse bereitzustellen, das den Wirkstoff erst nach einer vorherbestimmbaren Zeit nach der Applikation freisetzt.

Überraschenderweise hat sich gezeigt, daß die Aufgabe dadurch gelöst wird, daß die bei Körpertemperatur erweichende Reservoirschicht durch eine Vorrichtung aus für den Wirkstoff undurchlässigem Material von einer steuernden Membran beabstandet ist, die sich über die gesamten einander zugewandten Flächen des Reservoirs und der Membran erstreckt und mindestens einen Durchlaß für die bei Körpertemperatur erweichende Reservoirschicht aufweist.

Das wirkstoffhaltige Pflaster zur kontrollierten Abgabe von Wirkstoffen besteht demnach aus einer Rückschicht, einer daran angrenzenden, bei Körpertemperatur erweichenden, wirkstoffhaltigen Reservoirschicht, einer die Wirkstofffreisetzung steuernden Membran, einer die Fixierung des Pflasters auf der Haut ermöglichenden Haftklebevorrichtung und einer wiederablösbaren Schutzschicht, wobei die bei Körpertemperatur erweichende Reservoirschicht durch eine erfindungsgemäß ausgebildete Vorrichtung aus einem für den Wirkstoff undurchlässigen Material von der steuernden Membran beabstandet ist.

Das wirkstoffhaltige Reservoir hat die Aufgabe, den Wirkstoff aufzunehmen. Nach der Applikation erweicht es und schafft so die Möglichkeit, daß der Wirkstoff durch die Vorrichtung zur Einstellung des Abstandes zur Membran durchtritt.

Die Vorrichtung zur Einstellung des Abstandes schafft einen räumlichen Abstand zwischen Reservoirschicht und Membran und verhindert so deren vorzeitigen Kontakt. Auf diese Weise wird verhindert, daß Wirkstoff unerwünscht schnell bzw. spontan an die Membran gelangt und die Freisetzung in Gang gesetzt wird.

Die Membran hat die Aufgabe, die Wirkstofffreisetzung zu steuern und sicherzustellen, daß die Freisetzung nach der Kinetik nullter Ordnung erfolgt.

Die Vorrichtung zur Einstellung des Abstandes erstreckt sich über die gesamten, einander zugewandten Flächen des Reservoirs und der Membran. Sie weist mindestens einen Durchlass für den bei Körpertemperatur erweichenden Reservoirinhalt auf und kann sowohl aus einem zusammenhängenden Gebilde als auch aus sich nicht berührenden Einzelteilen bestehen.

Dabei muß dafür gesorgt werden, daß mindestens ein Durchlass vorhanden ist und dieser ausreichend groß ist, damit die erforderliche Wirkstoffmenge an die Membran gelangt. Es können jedoch auch mehrere symmetrisch oder unsymmetrisch verteilte Durchlässe über die Vorrichtung verteilt sein. Diese Durchlässe können z.B. auch in Form von Schlitzen ausgebildet sein. In allen diesen Fällen bildet die Vorrichtung eine zusammenhängende Einheit.

Die Einstellung des Abstandes kann jedoch auch durch dreidimensionale Formkörper erzielt werden, wie z.B. Kugeln, Halbkugeln, Kalotten, Quader, Zylinder und Würfel.

Als Materialien, aus denen die Vorrichtung zur Einstellung des Abstandes bestehen kann, kommen in Frage Metalle, natürliche und/oder synthetische und/oder organische Polymere oder Glas. Diese Stoffe müssen für den Wirkstoff undurchlässig sein. Dazu zählen beispielsweise Proteine wie Kollagen, Elastin, Albumin und Kasein;
Polysaccharide wie Cellulose und Cellulosederivate, Stärke und Stärkederivate sowie Galactomannan, Chitin und Pektin; Polyethylen, Polypropylen, Polyester, Polyamid, Polyurethan, Polyisobutylen, Polyethylen-Acrylsäure-Copolymere, Polyacrylnitril, Polyvinylchlorid, Polyvinylidenchlorid, Polytetrafluorethylen, Styrol-Butadien-Styrol-Blockcopolymere, Styrol-Isopren-Styrol-Blockcopolymere, Styrol-Ethylen-Butylen-Styrol-Blockcopolymere, Silikone, Acrylnitrilbutadienstyrol-Kautschuk, Polycarbonat, Polymethylmethacrylat, Polyoxyethylen, Polyoximethylen, Polystyrol, Polyvinylalkohol, Polyvinylacetat;
Polykieselsäure, Silikate, Magnesium-Aluminium-Silikate, Bentonit.
Weitere Materialien für die Vorrichtung zur Einstellung des Abstandes können ausgewählt sein aus Gruppen textiler Flächengebilde, Papier, Folien und Schaumstoff. Dazu zählen beispielsweise Vliesstoffe, Gewebe, Gelege sowie Papier in unterschiedlichen Qualitäten und Folien aus verschiedenen genannten Polymeren und Metallen sowie Schaumstoffe, hergestellt aus bekannten Rohstoffen.

Die bei Körpertemperatur erweichende Reservoirschicht enthält Wirkstoffe. Dazu gehören z.B.:
Atenolol, Acineton, Acetylsalicylsäure, Aceclidin, Amfetaminil, Amfetamin, Amylnitrit, Apophedrin, Atebrin, Alprostadil, Azulen, Arecolin, Anethol, Amylenhydrat, Acetylcholin, Acridin, Adenosintriphosphorsäure, L-Äpfelsäure, Alimemazin, Allithiamin, Allyl-Isothiocyanat, Aminoethanol, Apyzin, Apiol, Azatadin, Alprenolol, Äthinazon, Batrafen, Betahistin, Biperiden, Beta-Acetyldigoxin, Bopindolol, Benzatropin, Bupranolol, Benclonidin, Buprenorphin, Bisnorephedrin, Butacetoluid, Benactyzin, Clonidin, Clemastin, Carazolol, Clembuterol, Campher, Colecalciferol, Chloralhydrat, Clemastin, Chlorobutanol, Capsaicin, Cyclopentamin, Clobutinol, Chamazulen, Codein, Chlorpromazin, Chinin, Chlorthymol, Cyclophosphamid, Cinchocain, Chlorambuzil, Chlorphenesin, Diclofenac, Diltiazen, Dihydroergotamin, Dihydrocristin, Dihydrotoxin, Dimenhydrinat, Diethylaminsalicylat, Digoxin, Dimethocain, Diethylethan, Divinylethan, Dexchlorpheniramin, Dinoproston, Dixyrazin, Etofenamat, Ethylen glykol-Monosalicylat, Ephedrin, Ethosuximid, Enallylpropymal, Emylcamat, Erytroltetranitrat, Emetin, Enfluran, Eucalyptol, Ethylmorphin, 5-Fluoruracil, Fentanyl, Fluanison, Fencarbamid, Glibenclamid, Gallopamil, Guajazulen, Hydromorphon, Heparin-prodrugs, Herzglykoside, Halothan, Hyoscyamin, Histamin, Hydroxycain, Hexylresorcin, Ibuprofen, Isosorbiddinitrat, Isosorbit-5-mononitrat, Indometacin, Isominil, Isoaminilcitrat, Jod, Jodoform, Ketotifen, Ketoprofen, L-Thyroxin, Levonorgestrel, Lobelin, Lidocain, Lopirin, Levamisol, Mosidomin, Metoclopramid, Metoprolol, Methamphetaminil, Midodrin, Metadon, Muscarin-Agonist, Methylprylon, Methylphenidat, Mephenesin, Methylephedrin, Meclastin, Methopromazin, Mesuximid, Menthol, Methoxyfluran, Methylpentynol, Metixen, Misoprostol, Nicotin, Nicardipin, Nitroglycerin, Nifedipin, Nicotinsäure-β-butoxyethylenester, Nonylsäurevanillylamid, Nadolol, Norethisteronacetat, Nikotin-Agonist, Nicethamid, Norpseudoephedrin, Oestradiol, Oxytetracain, Oxyprenolol, Oxyphenbutazon, Oxychinolin, Pilocarpin, Prazosin, Physostigmin, Pindolol, Propranolol, Prostaglandin, Pentagotin, Piroxamin, Piroxicam, Pinen, Prolintan, Procyclidin, Piperazin, Pivazid, Phensuximid, Procain, Phenidamin, Promethazin, Penetrazol, Profenamin, Perazin, Phenol, Pethidin, Prenylamin, Phenoxybenzamin, Ryosidin, Resochin, Selegelin, Soquinolol, Salbutamol, Scopolamin, Salicylsäureester, Spartein, Tamoxifen, Tizanidin, Testosteron, Tilidin, Theophyllin, Trimegestone, Trichlorethylen, Timolol, Trifluorperazin, Tetracain, Trimipramin, Tranycypromin, Trimethadion, Tybamat, Thymol, Thioridazin, Verapamil, Valproinsäure, Yohimbin, sowie weitere, dem Fachmann geläufige, über die Haut, eingeschlossenen Schleimhäute, aufnehmbare Wirkstoffe. Selbstverständlich ist diese Aufzählung nicht abschließend.

Besonders bevorzugt sind die Wirkstoffe Nitroglycerin, Nikotin, Oestradiol und seine pharmazeutisch unbedenklichen Ester, sowie Gestagene und ihre pharmazeutisch unbedenklichen Ester.

Die Vorrichtung zur Einstellung des Abstandes wird unmittelbar nach Erzeugen der Reservoirschicht mit dieser in Kontakt gebracht. Das kann z.B. durch Einlegen oder Aufkaschieren erfolgen. Dieser Vorgang kann ausgeführt werden unter gleichzeitiger Anwendung von Druck.

Die Vorrichtung zur Einstellung des Abstandes kann außerdem auf die Membran aufgebracht und danach mit der Reservoirschicht vereinigt werden. Das kann z.B. derart erfolgen, daß die Membran aus der Schmelze beschichtet oder extrudiert und danach mit der Vorrichtung zur Einstellung des Abstandes vereinigt wird.

Die steuernde Membran ist für den Wirkstoff durchlässig. Sie kontrolliert die Freisetzung des Wirkstoffes. Über Dicke und Zusammensetzung der Membran wird die Wirkstofffreisetzung gesteuert.

Sie besteht aus Stoffen, die für den jeweiligen Wirkstoff durchlässig sind. Das können beispielsweise Polymere wie Ethylenvinylacetat-Copolymer, Polyurethan, Polyethylen, Polyproplyen, Polyvinylalkohol, Polyvinylacetat sein. Diese Membranen können auch mikroporös sein. Sie weisen eine Schichtdicke von 0,01 bis 10 mm, bevorzugt 0,02 bis 0,3 mm auf.

Die steuernde Membran kann aus einer Haftklebeschicht gebildet sein. Verwendet werden können alle dem Fachmann bekannte Haftkleber.

Das wirkstoffhaltige Pflaster kann außerdem auf der von der Haut abgewandten Seite der Reservoirschicht ein elastisches Druckelement enthalten. Dieses Element hat die Funktion, einen Druck auf die Reservoirschicht in Richtung Hautoberfläche auszuüben und so den Kontakt zur Membran zu erhöhen.

Die bei Körpertemperatur erweichende, wirkstoffhaltige Reservoirschicht kann bestehen aus Polyethylenglykol, Polypropylenglycol-Ether, Polyvinylalkohol, Wachsen auf der Basis von Rizinusölderivaten, Fettalkoholen, Fettalkoholethern, Capryl-/Caprinsäure-Triglycerid, Glycerinmonocaprylat, Glycerinmonolaurat, Glycerinmonodicaprylat, mittelkettige Partialglyceride und Mischungen daraus.

Die bei Körpertemperatur erweichende, wirkstoffhaltige Reservoirschicht hat eine Dicke von 0,01 bis 1,0 mm.

Die Zeit, nach der die Wirkstofffreisetzung beginnt, läßt sich durch verschiedene Maßnahmen vorherbestimmen. So spielen außer der Vorrichtung zur Einstellung der Abstandes die Zusammensetzung der Reservoirschicht und die Auswahl der Membran eine wesentliche Rolle. Durch die Auswahl geeigneter Bestandteile läßt sich die Viskosität und damit auch das Fließverhalten der bei Körpertemperatur erweichenden Reservoirschicht vorherbestimmen. Auch das Material, aus dem die Membran besteht, und die Dicke derselben ermöglichen es, die Zeit, nach der die Freisetzung beginnt, vorherzubestimmen.

Die Materialien für die undurchlässige Rückschicht und die wiederablösbare Schutzschicht sind dem Fachmann bekannt (z.B. DE 38 43 239).

Auch eine Stützschicht ist dem Fachmann bekannt (z.B. DE 38 43 239). Die Verwendung einer Stützschicht kann aus produktionstechnischen Gründen angezeigt sein. Das ist dann der Fall, wenn die bei Körpertemperatur erweichende wirkstoffhaltige Reservoirschicht keine ausreichende innere Festigkeit besitzt, um maschinell weiterverarbeitet werden zu können.

Die Haftklebevorrichtung dient dem Fixieren des Pflasters auf der Haut. Sie kann geometrisch verschieden sein und das Pflaster vollflächig oder partiell überziehen. Die Haftklebevorrichtung kann beispielsweise aus einem Ring gebildet sein, der das wirkstoffhaltige Reservoir umschließt. Sie kann jedoch auch beispielsweise aus einem Haftkleber in Form von Punkten, Rauten, Streifen und Netzwerken gebildet sein.

Die Haftklebevorrichtung zum Fixieren auf der Haut wird aus den dem Fachmann bekannten Haftklebern gebildet. Diese Haftklebeschichten weisen eine Dicke von 0,01 bis 0,9 mm auf.

Ein Verfahren zur Herstellung des Pflasters erfolgt in mehreren Schritten:
Die bei Körpertemperatur erweichende, wirkstoffhaltige Reservoirschicht kann eine Lösung, eine Dispersion, eine Suspension oder eine Schmelze sein. Bevorzugt ist jedoch die Herstellung der Reservoirschicht aus der Schmelze.

Dazu werden die Einzelbestandteile in einem Wasserbad geschmolzen, der Wirkstoff zugegeben und die Mischung durch Rühren homogenisiert. Die resultierende, wirkstoffhaltige Reservoirmasse wird auf eine Stützschicht beschichtet, mit der Vorrichtung zur Einstellung des Abstandes und danach mit der Membran kaschiert. Aus dem so erhaltenen Laminat werden flächenförmige Gebilde ausgestanzt. Diese werden auf eine mit Haftkleber versehene Rückschicht aufgelegt und das Ganze mit einer weiteren Haftkleberschicht überzogen und danach mit einer wiederablösbaren, beidseitig mit Silikon beschichteten Schutzschicht abgedeckt. Daraus werden derart flächenförmige Gebilde ausgestanzt, daß die Haftklebeschicht das wirkstoffhaltige Reservoir allseitig überragt.

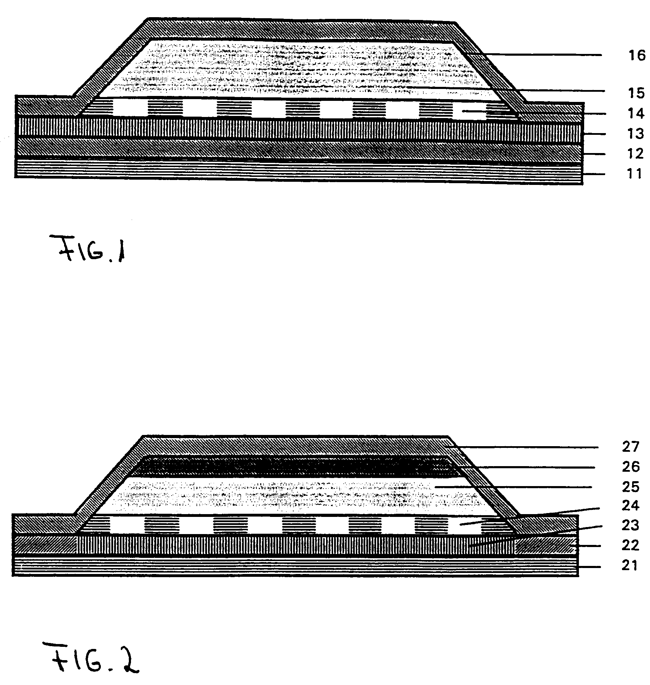

Die Erfindung wird anhand der Figuren näher erläutert. Die Figuren 1 und 2 zeigen nicht maßstabgerechte Querschnitte durch Pflaster gemäß der Erfindung.

In Figur 1 befindet sich der Wirkstoff in der bei Körpertemperatur erweichenden, wirkstoffhaltigen Reservoirschicht (15), die auf der Rückschicht (16) positioniert ist. Die im Kontakt mit dem Reservoir (15) befindliche Vorrichtung zur Einstellung des Abstandes mit mehreren Durchlässen ist mit (14) und die sich daran anschließende Membran mit (13) bezeichnet. Im Kontakt mit der Membran befindet sich eine vollflächige Haftklebevorrichtung (12), die mit der wiederablösbaren Schutzschicht (11) abgedeckt ist.

In Figur 2 befindet sich die bei Körpertemperatur erweichende, wirkstoffhaltige Reservoirschicht (25) im Kontakt mit dem elastischen Druckelement (26), das seinerseits auf der Rückschicht (27) positioniert ist. Die Vorrichtung zur Einstellung des Abstandes (24), wieder mit mehreren Durchlässen, befindet sich im Kontakt mit der Reservoirschicht (25) und ist auf der gegenüberliegenden Seite mit der Membran (23) verbunden. Im Kontakt mit der Membran (23) befindet sich die umlaufende Haftklebevorrichtung (22), die mit der wiederablösbaren Schutzschicht (21) abgedeckt ist.

### Beispiel 1:

Die bei Körpertemperatur erweichende Reservoirschicht wird wie folgt hergestellt:
- 350 g: Polyethylenglykol 600 (Fa. Merck)
- 150 g: Polyethylenglykol 1000 (Fa. Merck) werden bei 50°C im Wasserbad aufgeschmolzen und anschließend mit
- 500 g: Nitroglycerin-Lactose-Verreibung (10% Nitroglycerin - Fa. Dynamit Nobel)
versetzt und durch Rühren homogenisiert.

Die erhaltene nitroglycerinhaltige Reservoirmasse wird so auf eine Stützschicht aus Polyethylenterephthalat-Folie (Hostaphan RN 36 - Fa. Hoechst) beschichtet, daß eine wirkstoffhaltige Reservoirschicht mit einem Flächengewicht von ca. 225 g/m² resultiert. Auf diese Reservoirschicht wird dann als Vorrichtung zur Einstellung des Abstandes ein Vliesstoff (Paratex III/40 - Fa. Lohmann) und darauf eine Membran aus Ethylenvinylacetat-Copolymer (0,05 mm - MSP987192 - Fa. 3M) aufkaschiert.

Aus dem so erhaltenen Laminat aus Stützschicht, Reservoirschicht, Vorrichtung zur Einstellung des Abstandes und Membran werden Scheiben mit einer Fläche von 16 cm² ausgestanzt, auf die eine mit 20 g/m² Haftkleber auf Polyacrylatbasis (Durotak 280-2516 - Fa. National Starch) versehene Rückschicht (Hostaphan RN 15 - Fa. Hoechst) aufgelegt und das Ganze mit einer 100 g/m² Haftkleberschicht auf Polyacrylatbasis (Durotak 280-2516 - Fa. National Starch) überzogen und mit einer wiederablösbaren, beidseitig mit Silikon beschichteten Schutzschicht aus Polyethylenterephthalat (Hostaphan RN 100 - Fa. Hoechst) abgedeckt.

Daraus werden derart Scheiben gestanzt, daß die Haftkleberschicht das wirkstoffhaltige Reservoir allseitig um ca. 5 mm überragt.

Die Wirkstofffreisetzung wird wie folgt gemessen:

Das Wirkstoffpflaster wird in einem Schraubglas mit 80 ml physiologischer Kochsalzlösung bei 32°C (Hauttemperatur) geschüttelt, wobei Proben nach bestimmten Zeitabständen colorimetrisch vermessen werden.

### Beispiel 2:

Die wirkstoffhaltige Reservoirmasse wird, wie unter Beispiel 1 beschrieben, hergestellt, und ist wie folgt zusammengesetzt:
- 450 g: Glycerinester der Fettsäuren C₁₀ bis C₁₈ (Witepsol H 32 - Fa. Dynamit Nobel)
- 500 g: Nitroglycerin-Lactose-Verreibung (10% Nitroglycerin - Fa. Dynamit Nobel) und
- 50 g: Capryl-/Caprinsäure-Triglycerid (Miglyol 812 - Fa. Dynamit Nobel)

Das Wirkstoffpflaster wird, wie in Beispiel 1 beschrieben, hergestellt, und zwar unter Verwendung einer mikroporösen Membran aus Polyethylen, die schon mit der Vorrichtung zur Einstellung des Abstandes in Form eines Polypropylen-Vliesstoffes kombiniert ist (Gesamtdicke: 0,075 mm - Celgard 5550 - Fa. Celanese).

### Beispiel 3:

Die wirkstoffhaltige Reservoirschicht wird, wie in Beispiel 1 beschrieben, hergestellt, jedoch unter Verwendung der folgenden Rohstoffe:
- 450 g: Mischung von Tri- und Partialglyceriden der Fettsäuren C₈ bis C₁₈ (Softisan 601 - Fa. Dynamit Nobel)
- 500 g: Nitroglycerin-Lactose-Verreibung (10% Nitroglycerin - Fa. Dynamit Nobel) und
- 50 g: Capryl-/Caprinsäure-Triglycerid (Miglyol 812 - Fa. Dynamit Nobel).

Das Wirkstoffpflaster wird, wie in Beispiel 1 beschrieben, hergestellt, und zwar unter Verwendung einer mikroporösen Membran aus Polyethylen, die schon mit der Vorrichtung zur Einstellung des Abstandes kombiniert ist (Gesamtdicke: 0,13 mm - Celgard 5551 - Fa. Celanese).

Die Wirkstofffreisetzung des Pflasters gemäß Beispiel 1 ist nach 7 Stunden zu beobachten. Nach 24 Stunden wurden 0,82 mg Nitroglycerin freigesetzt.

Bei Beispiel 2 ist der erste Wirkstoffaustritt nach 3 Stunden zu beobachten, wobei nach 24 Stunden 2,21 mg Nitroglycerin freigesetzt wurden.

Bei Beispiel 3 beginnt die Wirkstofffreisetzung nach 3 Stunden. Nach 24 Stunden wurden 2,36 mg Nitroglycerin freigesetzt.
Bei einem mehrschichtigen nitroglycerinhaltigen Matrix-System gemäß DE 33 15 272 (Beispiel 1) dagegen beginnt die Wirkstofffreisetzung sofort.

## Patentansprüche

1. Wirkstoffhaltiges Pflaster zur kontrollierten Abgabe von Wirkstoffen aus einer Rückschicht, einer daran angrenzenden, bei Körpertemperatur erweichenden, wirkstoffhaltigen Reservoirschicht, einer die Wirkstofffreisetzung steuernden Membran, einer die Fixierung des Pflasters auf der Haut ermöglichenden Haftklebevorrichtung und einer wiederablösbaren Schutzschicht, dadurch gekennzeichnet, daß die bei Körpertemperatur erweichende Reservoirschicht durch eine Vorrichtung aus einem für den Wirkstoff undurchlässigen Material von der steuernden Membran beabstandet ist, die sich über die gesamten einander zugewandten Flächen des Reservoirs und der Membran erstreckt und mindestens einen Durchlass für die bei Körpertemperatur erweichende Reservoirschicht aufweist.

2. Wirkstoffhaltiges Pflaster nach Anspruch 1, dadurch gekennzeichnet, daß die Vorrichtung zur Einstellung des Abstandes aus einem zusammenhängenden Gebilde besteht.

3. Wirkstoffhaltiges Pflaster nach Anspruch 1 oder 2, dadurch gekennzeichnet, daß die Vorrichtung zur Einstellung des Abstandes aus sich nicht berührenden Einzelteilen besteht.

4. Wirkstoffhaltiges Pflaster nach einem oder mehreren der Ansprüche 1 bis 3, dadurch gekennzeichnet, daß die Vorrichtung zur Einstellung des Abstandes aus Metallen, natürlichen und/oder synthetischen anorganischen und/oder organischen Polymeren oder Glas besteht.

5. Wirkstoffhaltiges Pflaster nach einem oder mehreren der Ansprüche 1 bis 3, dadurch gekennzeichnet, daß die Vorrichtung zur Einstellung des Abstandes ausgewählt ist aus der Gruppe bestehend aus textilen Flächengebilden, Papier, Folien und Schaumstoff.

6. Wirkstoffhaltiges Pflaster nach Anspruch 3, dadurch gekennzeichnet, daß die Einzelteile der Vorrichtung zur Einstellung des Abstandes aus dreidimensionalen Formkörpern bestehen.

7. Wirkstoffhaltiges Pflaster nach Anspruch 1, dadurch gekennzeichnet, daß die bei Körpertemperatur erweichende Reservoirschicht bevorzugt Wirkstoffe, ausgewählt aus der Gruppe, bestehend aus Nitroglycerin, Nikotin und dessen pharmazeutisch akzeptablen Salzen, Oestrogene sowie Gestagene und deren pharmazeutisch akzeptablen Estern, enthält.

8. Wirkstoffhaltiges Pflaster nach Anspruch 1, dadurch gekennzeichnet, daß die steuernde Membran aus einer Haftklebeschicht gebildet ist.

9. Wirkstoffhaltiges Pflaster nach Anspruch 1, dadurch gekennzeichnet, daß sich auf der von der Haut abgewandten Seite der Reservoirschicht ein elastisches Druckelement befindet.

10. Verfahren zur Herstellung eines wirkstoffhaltigen Pflasters nach einem oder mehreren der vorangehenden Ansprüche, dadurch gekennzeichnet, daß die Vorrichtung zur Einstellung des Abstandes unmittelbar nach Erzeugen der Reservoirschicht mit dieser in Kontakt gebracht wird.

11. Verfahren zur Herstellung eines wirkstoffhaltigen Pflasters nach Anspruch 10, dadurch gekennzeichnet, daß die Vorrichtung zur Einstellung des Abstandes auf die steuernde Membran aufgebracht und danach mit der Reservoirschicht vereinigt wird.

## Claims

1. An active substance-containing patch for the controlled release of active substances, comprising a backing layer, an adjoining active substance-containing reservoir layer softening at body temperature, a membrane controlling the active substance release, a pressure-sensitive adhesive device permitting fixation of the patch to the skin, and a removable protective layer, characterized in that the reservoir layer which softens at body temperature is spaced away from the controlling membrane by means of a device made of a material impermeable to the active substance which extends all over the mutually facing surfaces of the reservoir and the membrane and which has at least one passage for the reservoir layer which softens at body temperature.

2. The active substance-containing patch according to claim 1 characterized in that the device for adjusting the distance consists of a coherent structure.

3. The active substance-containing patch according to claim 1 or 2 characterized in that the device for adjusting the distance consists of component parts not contacting one another.

4. The active substance-containing patch according to one or several of claims 1 to 3 characterized in that the device for adjusting the distance consists of metals, natural and/or synthetic inorganic and/or organic polymers, or of glass.

5. The active substance-containing patch according to one or several of claims 1 to 3 characterized in that the device for adjusting the distance is selected from the group consisting of textile fabrics, paper, films, and foam.

6. The active substance-containing patch according to claim 3 characterized in that the individual parts of the device for adjusting the distance consist of three-dimensional formed pieces.

7. The active substance-containing patch according to claim 1 characterized in that the reservoir layer softening at body temperature preferably comprises active substances selected from the group consisting of nitroglycerin, nicotine and its pharmaceutically acceptable salts, estrogens as well as gestagens and their pharmaceutically acceptable esters.

8. The active substance-containing patch according to claim 1 characterized in that the controlling membrane is formed of a pressure-sensitive adhesive layer.

9. The active substance-containing patch according to claim 1 characterized in that an elastic pressure element is present on the skin-averted side of the reservoir layer.

10. A process for the production of an active substance-containing patch according to one or several of the preceding claims, characterized in that the device for adjusting the distance is contacted with the reservoir layer immediately after formation thereof.

11. The process for the production of an active substance-containing patch according to claim 10 characterized in that the device for adjusting the distance is applied on the controlling membrane and then joined with the reservoir layer.

## Revendications

1. Emplâtre contenant des principes actifs, pour la libération contrôlée de principes actifs, constitué d'une couche dorsale, d'une couche de réservoir adjacente, contenant des principes actifs et ramollissant à température corporelle, d'une membrane contrôlant la libération des principes actifs, d'un dispositif de colle de contact permettant la fixation de l'emplâtre sur la peau et d'une couche de protection détachable, caractérisé en ce que la couche de réservoir ramollissant à température corporelle est séparée de la membrane de contrôle par un dispositif constitué d'un matériau imperméable au principe actif, dispositif qui s'étend sur toutes les surfaces du réservoir et de la membrane qui se trouvent face-à-face et en ce qu'il présente au moins un passage pour la couche de réservoir ramollissant à température corporelle.

2. Emplâtre contenant des principes actifs selon la revendication 1, caractérisé en ce que le dispositif pour le réglage de l'écartement est formé d'une structure cohérente.

3. Emplâtre contenant des principes actifs selon la revendication 1 ou 2, caractérisé en ce que le dispositif pour le réglage de l'écartement est constitué d'éléments individuels qui ne se touchent pas.

4. Emplâtre contenant des principes actifs selon une ou plusieurs des revendications 1 à 3, caractérisé en ce que le dispositif de réglage de l'écartement est constitué de métaux, de polymères naturels et/ou synthétiques, inorganiques et/ou organiques ou de verre.

5. Emplâtre contenant des principes actifs selon une ou plusieurs des revendications 1 à 3, caractérisé en ce que le dispositif de réglage de l'écartement est choisi parmi le groupe constitué d'une structure textile plane, de papier, de films ou de mousse.

6. Emplâtre contenant des principes actifs selon la revendication 3, caractérisé en ce que les éléments individuels du dispositif de réglage de l'écartement sont constitués d'objets moulés tridimensionnels.

7. Emplâtre contenant des principes actifs selon la revendication 1, caractérisé en ce que la couche de réservoir ramollissant à température corporelle contient de préférence des principes actifs choisis parmi le groupe constitué de la nitroglycérine, de la nicotine et de ses sels pharmaceutiquement acceptables, d'oestrogènes, de même que de gestagènes et de leurs esters pharmaceutiquement acceptables.

8. Emplâtre contenant des principes actifs selon la revendication 1, caractérisé en ce que la membrane de contrôle est formée d'une couche de colle de contact.

9. Emplâtre contenant des principes actifs selon la revendication 1, caractérisé en ce qu'un élément élastique exerçant une pression se trouve sur la face de la couche de réservoir opposée à la peau.

10. Procédé pour la préparation d'un emplâtre contenant des principes actifs selon une ou plusieurs des revendications précédentes, caractérisé en ce que le dispositif de réglage de l'écartement est mis en contact avec la couche de réservoir immédiatement après la création de celle-ci.

11. Procédé pour la préparation d'un emplâtre contenant des principes actifs selon la revendication 10, caractérisé en ce que le dispositif de réglage de l'écartement est appliqué sur la membrane de contrôle et ensuite combiné à la couche de réservoir.
